# EUROPEAN PATENT APPLICATION

(11) **EP 4 445 748 A1**
(43) Date of publication of application: **16.10.2024**
(21) Application number: 22904153.8
(22) Date of filing: 02.12.2022
(51) Int. Cl.: A23L 27/20, A23D 9/02, C12N 9/20, C12P 7/6409

(54) **ENZYME AGENT FOR IMPROVING FLAVOR, AND APPLICATION OF SAME**

(30) Priority: 07.12.2021 JP 2021198195
(71) Applicant: Amano Enzyme Inc., Nagoya-shi Aichi 460-8630 (JP)
(72) Inventor: ISHIGAKI, Yuki, Kakamigahara-shi, Gifu 509-0109 (JP)
(74) Representative: Witthoff Jaekel Steinecke Patentanwälte PartG mbB
(86) International application number: PCT/JP2022/044557
(87) International publication number: WO 2023/106224

(57) **Abstract**

It is an object of the present invention to provide a raw material, a food product, etc., which replace for dairy raw materials and have a good milk smell and a good milk flavor. The present invention relates to: a flavor-improving enzyme agent for vegetable oils and fats, including lipase; a flavor improver for food products, which is obtained by allowing lipase to act on vegetable oils and fats; and a food product, including the flavor improver for food products. In addition, the present invention relates to: a method for producing a flavor improver for food products, including a step of allowing lipase to act on vegetable oils and fats; and a method for producing a food product, including a step of allowing lipase to act on vegetable oils and fats to obtain a flavor improver for food products, and a step of obtaining a food product including the flavor improver for food products.

## Description

### TECHNICAL FIELD

The present invention relates to a flavor-improving enzyme agent, a flavor improver for food products, a food product, a method for producing a flavor improver for food products, and a method for producing a food product.

### BACKGROUND ART

Lipase is an enzyme that catalyzes a reaction of acting on triacylglycerol (triglyceride) to allow the triacylglycerol (triglyceride) to release fatty acid depending on the specificity of the enzyme, so as to produce diacylglycerol (DAG) or monoacylglycerol (MAG), or a reverse reaction thereof. It is known that lipase is used in concentration of EPA and DHA and production of enzyme-treated cheese and butter.

It is known that lipase is used in concentration of EPA and DHA and production of enzyme-treated cheese and butter. For example, Patent Document 1 discloses a method for producing a butter flavor, which is characterized in that it includes allowing pregastric esterase to act on a butter oil to decompose it, and then allowing pancreatic lipase or lipase produced by microorganisms belonging to Aspergillus sp., Mucor sp. or Rhizopus sp. to further act on the butter oil to decompose it. Herein, it has been studied regarding allowing lipase to act on a butter oil as animal oils and fats, so as to provide a butter oil with enhanced residual aroma obtained when it is heated.

Moreover, Patent Document 2 discloses a technique of improving the rich taste of oils and fats by adding a virgin coconut oil to the fats and oils. Furthermore, Patent Document 3 discloses a method of hydrolyzing a coconut oil using lipase. In Patent Document 3, a technique of pressure decomposition method for oils and fats, which has been modified so that the induction period is substantially eliminated, has been proposed.

### PRIOR ART DOCUMENTS

### PATENT DOCUMENTS

Patent Document 1: JP Patent Publication No. S59-66856 A (1984)
Patent Document 2: International Publication WO2016/136688
Patent Document 3:JP Patent Publication No. H8-507917 A (1996)

### SUMMARY OF INVENTION

### OBJECTS TO BE SOLVED BY THE INVENTION

As disclosed in Patent Document 1, a technique of allowing lipase to act on dairy raw materials to enhance the flavor, etc. has been known. However, in recent years, with the spread of SDGs and an increase in vegan population, the replacement of dairy raw materials by vegetable-derived raw materials has been considered. Accordingly, it has been desired to establish a technique of enhancing a milk flavor without using dairy raw materials derived from animals. Besides, Patent Document 3 discloses a technique of pressure decomposition method for oils and fats, in which a coconut oil is hydrolyzed using lipase. However, in Patent Document 3, application of the materials obtained by this pressure decomposition method for oils and fats to food products has not been studied, and the flavor, etc. has not been evaluated, either.

Thus, it is an object of the present invention to provide a raw material and a food product that replace for dairy raw materials and have a good milk smell, a good milk flavor, etc.

### MEANS FOR SOLVING THE Objects

As a result of intensive studies conducted directed towards achieving the aforementioned object, the present inventors have found that a milk smell and a milk flavor can be imparted to vegetable oils and fats by allowing lipase to act on the vegetable oils and fats, thereby completing the present invention.

Specifically, the present invention has the following configurations.

[1] A flavor-improving enzyme agent for vegetable oils and fats, which includes lipase.
[2] The flavor-improving enzyme agent according to [1], in which the lipase is at least one type selected from the group consisting of a lipase derived from Penicillium sp., a lipase derived from Aspergillus sp., and a lipase derived from Mucor sp.
[3] The flavor-improving enzyme agent according to [1] or [2], in which the vegetable oils and fats are coconut oil.
[4] A flavor improver for food products, which is obtained by allowing lipase to act on vegetable oils and fats.
[5] The flavor improver for food products according to [4], in which the amount of free fatty acid contained in the flavor improver for food products is 0.6 to 500 mg per mL of the vegetable oils and fats.
[6] The flavor improver for food products according to [4] or [5], in which the lipase is at least one type selected from the group consisting of a lipase derived from Penicillium sp., a lipase derived from Aspergillus sp., and a lipase derived from Mucor sp.
[7] The flavor improver for food products according to any one of [4] to [6], in which the vegetable oils and fats are coconut oil.
[8] A food product, including the flavor improver for food products according to any one of [4] to [7].
[9] The food product according to [8], in which the amount of free fatty acid contained in the food product is 0.1 to 60 mg per mL of the vegetable oils and fats.
[10] A method for producing a flavor improver for food products, including a step of allowing lipase to act on vegetable oils and fats.
[11] The method for producing a flavor improver for food products according to [10], in which the lipase is at least one type selected from the group consisting of a lipase derived from Penicillium sp., a lipase derived from Aspergillus sp., and a lipase derived from Mucor sp.
[12] The method for producing a flavor improver for food products according to [10] or [11], in which the vegetable oils and fats are coconut oil.
[13] The method for producing a flavor improver for food products according to any one of [10] to [12], in which the amount of free fatty acid contained in the flavor improver for food products is 0.6 to 500 mg per mL of the vegetable oils and fats.
[14] A method for producing a food product, including:
   allowing lipase to act on vegetable oils and fats to obtain a flavor improver for food products, and
   obtaining a food product including the flavor improver for food products.
[15] The method for producing a food product according to [14], in which the lipase is at least one type selected from the group consisting of a lipase derived from Penicillium sp., a lipase derived from Aspergillus sp., and a lipase derived from Mucor sp.
[16] The method for producing a food product according to [14] or [15], in which the vegetable oils and fats are coconut oil.
[17] The method for producing a food product according to any one of [14] to [16], in which the amount of free fatty acid contained in the food product is 0.1 to 60 mg per mL of the vegetable oils and fats.

In addition, the present invention has the following configurations.
[A] A method of improving a flavor, including allowing lipase to act on vegetable oils and fat.
[B] An enzyme agent including lipase, for use in improving the flavor of vegetable oils and fats.
[C] Use of an enzyme agent including lipase, for improving the flavor of vegetable oils and fats.
[D] Use of an enzyme agent including lipase, for improving the flavor of a food product.

### Advantageous EFFECTS OF INVENTION

According to the present invention, there can be obtained a flavor-improving enzyme agent, which allows lipase to act on vegetable oils and fats, so that it can impart a milk smell and a milk flavor to the vegetable oils and fats. In addition, according to the present invention, there can be provided a flavor improver for food products, a food product, and the like, which exhibit a milk smell and a milk flavor.

### EMBODIMENTS OF CARRYING OUT THE INVENTION

Hereafter, the present invention will be described in detail. The configuration requirements described below may be described based on representative embodiments or specific examples, but the present invention is not limited to such embodiments. In the present description, a numerical value range expressed using the preposition "to" means a range that includes the numerical values described before and after the preposition "to" as lower and upper limits.

### (Flavor-improving enzyme agent)

The present invention relates to a flavor-improving enzyme agent for vegetable oils and fats, which includes lipase. The flavor-improving enzyme agent is not particularly limited, as long as it is an agent including lipase. The flavor-improving enzyme agent may also be an agent consisting of lipase. Moreover, the flavor-improving enzyme agent may be in a powder, solid, gel, or liquid form. In the present invention, the flavor-improving enzyme agent is allowed to act on vegetable oils and fats, so that a milk smell and a milk flavor can be imparted to the vegetable oils and fats. In the present description, the milk smell refers to a milk-like aroma, and the milk flavor refers to milk-like taste and aroma. By treating vegetable oils and fats with the flavor-improving enzyme agent, such a milk smell and/or a milk flavor can be imparted to the vegetable oils and fats, and as a result, vegetable-derived raw materials can be replaced for dairy raw materials.

The present embodiment may relate to a method of improving the flavor of vegetable oils and fats using a flavor-improving enzyme agent. The flavor-improving method includes a step of allowing lipase to act on vegetable oils and fats. According to the flavor-improving method, fatty acid can be released from triacylglycerol (triglyceride) included in the obtained vegetable oils and fats, and thereby, the vegetable oils and fats can exhibit a milk smell and/or a milk flavor.

### <Lipase>

Lipase is an enzyme that catalyzes a reaction of acting on triacylglycerol (triglyceride) to allow the triacylglycerol (triglyceride) to release fatty acid depending on the specificity of the enzyme, so as to produce diacylglycerol (DAG) or monoacylglycerol (MAG), or a reverse reaction thereof. As long as the above-described action is a main activity, the type, origin, etc. of lipase are not particularly limited, and the flavor-improving enzyme agent may include components other than the lipase.

Examples of the lipase may include a lipase derived from Penicillium sp., a lipase derived from Aspergillus sp., a lipase derived from Rhizopus sp., a lipase derived from Candida sp., a lipase derived from Mucor sp., a lipase derived from Rhizomucor sp., and a lipase derived from Penicillium sp. These lipases may be used alone as a single type, or may also be used in combination of multiple types. Among others, from the viewpoint of the improvement of the flavor of a food product, the lipase is preferably at least one type selected from the group consisting of a lipase derived from Penicillium sp., a lipase derived from Aspergillus sp., and a lipase derived from Mucor sp.; and is more preferably at least one type selected from the group consisting of a lipase derived from *Aspergillus niger* and a lipase derived from Penicillium sp.; and is further preferably a lipase derived from Penicillium sp. It is to be noted that when the lipase is at least one type selected from the group consisting of a lipase derived from *Aspergillus niger* and a lipase derived from Penicillium sp., the amount of released fatty acid tends to hardly increase to a predetermined amount or more, and thus, such lipase is suitable for intended uses where the reaction is desirably terminated at a certain degradation level.

Lipase can be prepared from a culture medium of microorganisms serving as an origin of lipase as mentioned above. A specific preparation method may be a method of recovering lipase from the culture medium or cell mass of the above-described microorganisms. For example, in the case of using lipase-secreting microorganisms, the cell mass has previously been recovered from the culture medium by filtration, centrifugation, etc., as necessary, and the enzyme is then separated and/or purified. In the case of using lipase-non-secreting microorganisms, the cell mass has previously been recovered from the culture medium, as necessary, and the recovered cell mass is then crushed by a pressure treatment, an ultrasonic treatment, etc. to expose the enzyme, and the enzyme is then separated and/or purified. The method of separating and/or purifying the enzyme is not particularly limited, and a known protein separation and/or purification method can be adopted. Examples of such a protein separation and/or purification method may include a centrifugal separation method, a UF concentration method, a salting-out method, and various types of chromatographic methods using ion exchange resin, etc. The separated and/or purified enzyme can be powderized by drying methods such as freeze-drying and drying under reduced pressure. Otherwise, the separated and/or purified enzyme can also be powderized by using suitable excipients and/or drying aids in such drying methods. Moreover, the separated and/or purified enzyme can also be liquefied by adding suitable additives thereto and performing filter sterilization.

A commercially available product can also be used as such lipase. Examples of a preferred commercially available product may include Lipase R "Amano" (derived from *Penicillium rokkforitii*) and Lipase A "Amano" 6 (derived from *Aspergillus niger*)*,* which are manufactured by Amano Enzyme Inc.

When the flavor-improving enzyme agent is allowed to act on vegetable oils and fats, the amount of lipase allowed to act thereon is not particularly limited. For example, the lipase is preferably added, so that the enzyme activity becomes 0.01 U or more with respect to 1 g of the vegetable oils and fats. From the viewpoint of further enhancing the flavor-improving effects, the enzyme activity is more preferably 0.05 U or more, further preferably 0.1 U or more, still further preferably 0.8 U or more, and particularly preferably 1.5 U or more, with respect to 1 g of the vegetable oils and fats. On the other hand, the upper limit value of the enzyme activity is not particularly limited, and for example, the upper limit value is preferably 500 U or less, more preferably 100 U or less, further preferably 50 U or less, even further preferably 40 U or less, still further preferably 30 U or less, and particularly preferably 22 U or less, with respect to 1 g of the vegetable oils and fats. It is to be noted that the additive amount of the flavor-improving enzyme agent is preferably adjusted, as appropriate, according to the type of lipase.

The enzyme activity of lipase is measured by the following method. First, 22.5 g of olive oil is mixed with 75 mL of emulsion (18 g/L polyvinyl alcohol I and 2 g/L polyvinyl alcohol II), and the mixture is then emulsified using a homogenizer to prepare a substrate solution. To 5 mL of the substrate solution and 4 mL of McIlvaine buffer (pH 7.0), 1 mL of a lipase solution is added, followed by performing a reaction at 37°C. Thirty minutes later, 10 mL of an ethanol-acetone mixed solution is added to the reaction mixture to terminate the enzyme reaction. Subsequently, 10 mL of a 0.05 mol/L sodium hydroxide solution and 10 mL of an ethanol-acetone mixed solution are added to the reaction mixture, and titration is carried out with 0.05 mol/L hydrochloric acid, so that the pH value becomes 10. The amount of the enzyme that causes an increase of 1 µmol of fatty acids per minute is defined as one unit (U).

### <Vegetable oils and fats>

The vegetable oils and fats used herein are not particularly limited in terms of the origin, properties, etc. Examples of the vegetable oils and fats may include soybean oil, rapeseed oil, corn oil, sesame oil, Japanese basil oil, linseed oil, peanut oil, safflower oil, high oleic safflower oil, sunflower oil, high oleic sunflower oil, cottonseed oil, grape seed oil, macadamia nut oil, hazelnut oil, pumpkin seed oil, walnut oil, camellia oil, tea seed oil, perilla oil, borage oil, olive oil, rice bran oil, wheat germ oil, coconut oil, cocoa oil, palm oil, palm kernel oil, and algae oil. These vegetable oils and fats may be used alone or may also be used in combination of two or more types. Among others, the vegetable oils and fats are preferably coconut oils.

Regarding the properties of the vegetable oils and fats subjected to the treatment with the flavor-improving enzyme agent, the vegetable oils and fats may be, for example, a liquid, a slurry, or a paste.

### (Flavor improver for food products)

The present invention relates to a flavor improver for food products, which is obtained by allowing lipase to act on vegetable oils and fats. The flavor improver for food products is an oil and fat composition obtained by treating vegetable oils and fats with lipase. The flavor improver for food products has a milk smell and/or a milk flavor by itself. Therefore, by adding the flavor improver for food products to a food product, a milk smell and/or a milk flavor can be imparted to the food product. In addition, the food product, to which the flavor improver for food products has been added, can exhibit a milk smell and/or a milk flavor without using dairy raw materials, and it also becomes possible to provide a milk-flavored food product that is free from animal-derived raw materials.

The lipase and the vegetable oils and fats, as mentioned above, are exemplified as a lipase and vegetable oils and fats used in the production of the flavor improver for food products.

By allowing lipase to act on vegetable oils and fats, fatty acid is released from triacylglycerol (triglyceride) included in the vegetable oils and fats. Therefore, the flavor improver for food products includes the released fatty acids. The amount of free fatty acid included in the flavor improver for food products is preferably 0.6 mg or more, more preferably 0.7 mg or more, further preferably 1.0 mg or more, even further preferably 2.0 mg or more, still further preferably 3.0 mg or more, and particularly preferably 4.0 mg or more, per mL of the vegetable oils and fats. On the other hand, the amount of free fatty acid included in the flavor improver for food products is preferably 500 mg or less, more preferably 400 mg or less, further preferably 300 mg or less, even further preferably 200 mg or less, still further preferably 100 mg or less, and particularly preferably 75 mg or less, per mL of the vegetable oils and fats. The above-described amount of free fatty acid can be adjusted by changing the type of the lipase or conditions for the lipase treatment. Moreover, the above-described amount of free fatty acid can also be adjusted by mixing two or more types of oils and fats having each different fatty acid content with each other. By adjusting the amount of free fatty acid included in the flavor improver for food products within the above-described range, a flavor improver for food products having a good milk smell or a good milk flavor can be obtained.

Besides, when the amount of free fatty acid in the flavor improver for food products is a predetermined amount or more, a pungent smell may be exhibited more strongly than a milk smell and a milk flavor in some cases. In such a case, the vegetable oils and fats may be mixed with vegetable oils and fats or other components, containing a small amount of free fatty acid, so that the amount of free fatty acid can be adjusted within the above-described range, and thereby, a flavor improver for food products exhibiting a good milk smell or a good milk flavor can be obtained. Hence, the present embodiment may also relate to a concentrated flavor improver for food products, in which the amount of free fatty acid exceeds the aforementioned upper limit value. When such a concentrated flavor improver for food products is used, it is mixed with vegetable oils and fats or with other components, containing a small amount of free fatty acid, or it is mixed with food raw materials upon production of a food product, so that a flavor improver for food products or a food product, exhibiting a good milk smell or a good milk flavor, can be obtained.

### (Method for producing flavor improver for food products)

The present invention relates to a method for producing a flavor improver for food products, including a step of allowing lipase to act on vegetable oils and fats. In the present method for producing a flavor improver for food products, vegetable oils and fats are treated with lipase, so that fatty acid is released from triacylglycerol (triglyceride) included in the vegetable oils and fats. Thereby, a flavor improver for food products exhibiting a milk smell or a milk flavor can be obtained.

The lipase and the vegetable oils and fats, as mentioned above, are exemplified as a lipase and vegetable oils and fats used in the production of the flavor improver for food products. In addition, the amount of free fatty acid included in the produced flavor improver for food products is preferably within the above-mentioned range.

The reaction time, temperature, pH of the reaction solution, and the like, which are applied for allowing lipase to act on vegetable oils and fats, are not particularly limited. The reaction temperature is, for example, preferably 20°C to 80°C, more preferably 30°C to 70°C, and further preferably 40°C to 60°C. The pH of the reaction solution is, for example, preferably pH 4 to 10, more preferably pH 5 to 9, and further preferably pH 6 to 8. The reaction time is, for example, preferably 10 minutes to 240 hours, more preferably 1 to 96 hours, and further preferably 2 to 48 hours. By adopting the aforementioned reaction conditions, vegetable oils and fats with a good a milk smell and a good milk flavor can be easily obtained. It is to be noted that these reaction conditions are selected, as appropriate, depending on a flavor improver for food products of interest. The optimal reaction conditions may be determined through preliminary experiments, etc.

The method for producing a flavor improver for food products may include a step of inactivating lipase, after the step of allowing the lipase to act on the vegetable oils and fats. In the step of inactivating lipase, for example, the vegetable oils and fats treated with the lipase can be heated to 80°C or higher, or the enzyme solution can be separated from the oils and fats by centrifugation.

### (Food product)

The flavor improver for food products can be used as a food product by itself, or it can also be used as a flavor improver in raw materials of other food products. The present invention also relates to a food product including the aforementioned flavor improver for food products. Examples of the food product including the flavor improver for food products may include vegetable-based alternative food products, and may specifically be vegetable-based dairy products. Specific examples of the vegetable-based dairy products may include alternative cheese, alternative butter, and alternative milk. The vegetable-based dairy product is preferably alternative butter, and the vegetable-based dairy products may be food products including alternative cheese, alternative butter, alternative milk, etc. Since these food products include the flavor improver for food products, a milk smell or a milk flavor is imparted thereto.

Since the food product of the present invention includes the flavor improver for food products, fatty acid is included in the food product. The amount of free fatty acid included in the present food product is preferably 0.1 mg or more, more preferably 0.2 mg or more, even more preferably 0.3 mg or more, further preferably 0.5 mg or more, even further preferably 1.0 mg or more, still further preferably 2.0 mg or more, particularly preferably 3.0 mg or more, and most preferably 4.0 mg or more, per mL of the vegetable oils and fats. On the other hand, the amount of free fatty acid included in the present food product is preferably 60 mg or less, more preferably 40 mg or less, further preferably 30 mg or less, and particularly preferably 20 mg or less, per mL of the vegetable oils and fats. The above-described amount of free fatty acid can be adjusted by changing the type of the lipase or conditions for the lipase treatment. Moreover, the above-described amount of free fatty acid can also be adjusted by mixing two or more types of oils and fats having each different fatty acid content into the food product. By adjusting the amount of free fatty acid included in the food product within the above-described range, a food product having a good milk smell or a good milk flavor can be obtained.

### (Method for producing food product)

The present invention relates to a method for producing a food product, including a step of allowing lipase to act on vegetable oils and fats to obtain a flavor improver for food products, and a step of obtaining a food product including the flavor improver for food products. In the present method for producing a food product, in the step of obtaining a flavor improver for food products, vegetable oils and fats are treated with lipase, so that fatty acid is released from triacylglycerol (triglyceride) included in the vegetable oils and fats. Then, the flavor improver for food products including the released fatty acid is added to a food raw material, so that a food product exhibiting a milk smell or a milk flavor can be obtained.

The lipase and the vegetable oils and fats, as mentioned above, are exemplified as a lipase and vegetable oils and fats used in the production of the food product. In addition, the amount of free fatty acid included in the produced food product is preferably within the above-mentioned range.

As reaction conditions applied in the step of allowing lipase to act on vegetable oils and fats to obtain a flavor improver for food products, the above-mentioned conditions are preferably adopted. Moreover, the method for producing a food product may include a step of inactivating lipase, after the step of allowing the lipase to act on the vegetable oils and fats to obtain a flavor improver for food products, or after the step of obtaining a food product including the flavor improver for food products.

In the step of obtaining a food product including the flavor improver for food products, it is preferable that a flavor improver for food products including the released fatty acid is added to mixed with a food raw material. At this time, the additive amount of the flavor improver for food products is desirably adjusted, so that the amount of free fatty acid included in the food product becomes an appropriate range. It is to be noted that the food raw material may include or may not include oils and fats.

### EXAMPLES

Hereinafter, the characteristics of the present invention will be more specifically described in the following examples. The materials, amounts used, proportions, treatment contents, treatment procedures, etc. shown in the following examples may be changed, as appropriate, without deviating from the gist of the present invention. Therefore, the scope of this invention should not be interpreted limitedly by the specific examples shown below.

### <Measurement methods>

### <Method of measuring lipase activity>

Olive oil (22.5 g) was mixed with 75 mL of emulsion (18 g/L polyvinyl alcohol I and 2 g/L polyvinyl alcohol II), and the mixture was then emulsified using a homogenizer to prepare a substrate solution. To 5 mL of the substrate solution and 4 mL of McIlvaine buffer (pH 7.0), 1 mL of a lipase solution was added, followed by performing a reaction at 37°C. Thirty minutes later, 10 mL of an ethanol-acetone mixed solution was added to the reaction mixture to terminate the enzyme reaction. Subsequently, 10 mL of a 0.05 mol/L sodium hydroxide solution and 10 mL of an ethanol-acetone mixed solution were added to the reaction mixture, and titration was then carried out with 0.05 mol/L hydrochloric acid, so that the pH value became 10. The amount of the enzyme that causes an increase of 1 µmol of fatty acids per minute was defined as one unit (U).

### <Method of measuring amount of fatty acid (amount of free fatty acid)>

After a lipase treatment, 30 µL of oils and fats was dissolved in 1 mL of hexane, which was then subjected to Gas Chromatography GC-2010 Plus, manufactured by Shimadzu Corporation. In a case where the amount of fatty acid was not measured in oils and fats alone (i.e. in the case of measuring the amount of fatty acid in a food product), the oil and fat component was extracted with the organic solvent diethyl ether or hexane, and then the aforementioned operation was carried out. The conditions for gas chromatography were as follows. Detector: FID; column: DB-1701 (15 m, 25 mm, and 0.15 µm); split ratio: 20; and oven conditions: 60°C, temperature increased to 250°C at 10°C/min and maintained at 250°C for 10 minutes. The amount of fatty acid in the sample was calculated by comparison with the analysis results (area) of 1 mg/mL of each fatty acid reagent.

### <Test examples>

### <Examples 1 to 13 and Comparative Example 1: Oil and Fat A>

### (1) Experimental method

Coconut oil (20 g) was poured into a beaker, and 1 mL of a lipase solution having a predetermined lipase concentration was added thereto (the lipase solution was added so that the water content became 5% with respect to the substrate oil and fat). The obtained mixture was reacted at 50°C overnight. In each example, as lipases, Lipase A "Amano" *6 (Aspergillus* niger-derived lipase), Lipase MH "Amano" 10SD (derived from *Mucor Javanicus*)*,* and Lipase R "Amano" (derived from *Penicillium roqueforti*), which were manufactured by Amano Enzyme Inc., were used, as shown in the following table. In Comparative Example 1, a lipase solution was not added.

### (2) Results

The enzyme activity of the added lipase and the amount of free fatty acid after the enzyme treatment were measured. In addition, the obtained enzyme-treated oils and fats (flavor improvers for food products) were evaluated in terms of the presence or absence of a milk smell. These results are shown in Table 1 below.

**[Table 1]**

| | | Activity of enzyme added (U/g oils and fats) | Amount of free fatty acid (mg/mL) | Milk smell |
|---|---|---|---|---|
| Comp. Ex. 1 | Not added | | 0.6 | - |
| Ex. 1 | Lipase A "Amano" 6 | 12.9 U | 7.8 | A |
| Ex. 2 | | 17.2 U | 6.2 | A |
| Ex. 3 | | 21.5 U | 9.4 | A |
| Ex. 4 | Lipase MH "Amano" 10SD | 0.13 U | 2.8 | A |
| Ex. 5 | | 0.65 U | 55.0 | A |
| Ex. 6 | | 1.3 U | 62.9 | B |
| Ex. 7 | | 13.0 U | 221.5 | B* |
| Ex. 8 | | 26.0 U | 281.8 | B* |
| Ex. 9 | Lipase R "Amano" | 0.9 U | 14.2 | A |
| Ex. 10 | | 1.8 U | 24.8 | A |
| Ex. 11 | | 2.7 U | 19.3 | A |
| Ex. 12 | | 3.6 U | 22.7 | A |
| Ex. 13 | | 4.5 U | 28.5 | A |

A: Oil and fat, in which a milk smell was felt compared with the control
B:Oil and fat, in which a pungent smell as well as a milk smell was felt compared with the control
B*: Oil and fat, in which a milk smell was felt and a pungent smell was strongly felt, compared with the control
-: Oil and fat, in which no milk smell was felt

### (3) Consideration

From the enzyme-treated coconut oil, a milk smell was felt, compared with the enzyme treatment-free coconut oil. From the coconut oils obtained in Examples 7 and 8, a strong pungent smell was felt, which was considered to the result that the milk smell became excessively strong.

### <Examples 14 and 15: Oil and Fat B>

### (1) Experimental method

The enzyme-treated coconut oil obtained in Example 7 was appropriately diluted with the enzyme treatment-free coconut oil (Examples 14 and 15), and a milk smell was then evaluated.

### (2) Results

The results are shown in Table 2 below.

**[Table 2]**

| | Mixing ratio | | Estimated amount of free fatty acid (mg/mL) | Milk smell |
|---|---|---|---|---|
| | Lipase-treated coconut oil | Enzyme treatment-free coconut oil | | |
| Comp. Ex. 1 | 0 | 10 | 0.6 | - |
| Ex. 14 | 1 | 9 | 22.7 | A |
| Ex. 15 | 2 | 8 | 44.8 | A |
| Ex. 7 | 10 | 0 | 221.5 | B* |

A: Oil and fat, in which a milk smell was felt compared with the control
B*: Oil and fat, in which a milk smell was felt and a pungent smell was strongly felt, compared with the control
-: Oil and fat, in which no milk smell was felt

### (3) Consideration

It was revealed that the lipase-treated coconut oil having a pungent smell exhibits a moderate milk smell, if it is diluted with a lipase treatment-free coconut oil.

### <Examples 16 to 21 and Comparative Example 2: Oil and Fat C>

### (1) Experimental method

Coconut oil (20 g) was poured into a beaker, and 1 mL (w/v) of a 20 mg/mL lipase solution was added thereto. The obtained mixture was reacted at 50°C, and the amount of free fatty acid was measured every predetermined time. As lipases, Lipase A "Amano" 6 *(Aspergillus* niger-derived lipase) and Lipase R "Amano" (derived from *Penicillium roqueforti*), which were manufactured by Amano Enzyme Inc., were used. In

Comparative Example 2, a lipase solution was not added.

### (2) Results

The results are shown in Table 3 below.

**[Table 3]**

| | | Activity of enzyme added (U/g oils and fats) | Treatment time (hour) | Amount of free fatty acid (mg/mL) | Milk smell |
|---|---|---|---|---|---|
| Comp. Ex. 2 | Not added | | | 0.5 | - |
| Ex. 16 | Lipase A "Amano" 6 | 4.3 U | 2 | 4.2 | A |
| Ex. 17 | | | 6 | 4.1 | A |
| Ex. 18 | | | 22 | 4.6 | A |
| Ex. 19 | Lipase R "Amano" | 0.9 U | 2 | 15.9 | A |
| Ex. 20 | | | 6 | 14.4 | A |
| Ex. 21 | | | 22 | 14.1 | A |

A: Oil and fat, in which a milk smell was felt compared with the control
-: Oil and fat, in which no milk smell was felt

### (3) Consideration

The amount of free fatty acid could be adjusted by adjusting the amount of the enzyme added and the reaction time. It was found that when Aspergillus-derived lipase (Lipase A "Amano" 6) or Penicillium-derived lipase (Lipase R "Amano") is used, the amount of free fatty acid is hardly increased due to the properties of these enzymes, and thus that these enzymes are easily used for intended uses where the reaction is desirably terminated at a certain degradation level.

### <Examples 22 to 25: Plant-based dairy products>

### (1) Experimental method

Coconut oil treated with Lipase R "Amano" (the amount of free fatty acid was 3 mg/mL per mL of the oil and fat) or coconut oil treated with Lipase MH "Amano" (the amount of free fatty acid was 199 mg/mL per mL of the oil and fat) was added in an amount of 1% by mass to a commercially available vegetable food product, and the obtained food product was then evaluated in terms of a milk smell.

### (2) Results

The results are shown in Table 4 below.

**[Table 4]**

| | Vegetable food product | Treating enzyme | Amount of free fatty acid (mg/mL) | Milk smell |
|---|---|---|---|---|
| Ex. 22 | Soy milk | Coconut oil treated with Lipase R "Amano" | 0.75 | A |
| Ex. 23 | Oat milk | | 1.2 | A |
| Ex. 24 | Margarine | Coconut oil treated with Lipase MH "Amano" | 2.3 | A |
| Ex. 25 | Vegetable cheese | | 34.9 | A |
| Ex. 26 | Vegetable cream | | 4.7 | A |

A: Food product, in which a milk smell was more strongly felt than a food product to which an enzyme-treated coconut oil was not added

### <Example 27 and Comparative Examples 3 and 4: Cookies>

### (1) Experimental method

Cookies were prepared by adding a coconut oil treated with Lipase R "Amano" (the amount of free fatty acid was 3 mg/mL per mL of the oil and fat) to materials. After the materials shown in Table 5 below had been mixed with one another, the obtained mixture was baked in an oven at 180°C for 20 minutes. Thereafter, the prepared cookies were eaten to check the flavor (milk flavor) of the cookies.

### (2) Results

The results are shown in Table 5 below.

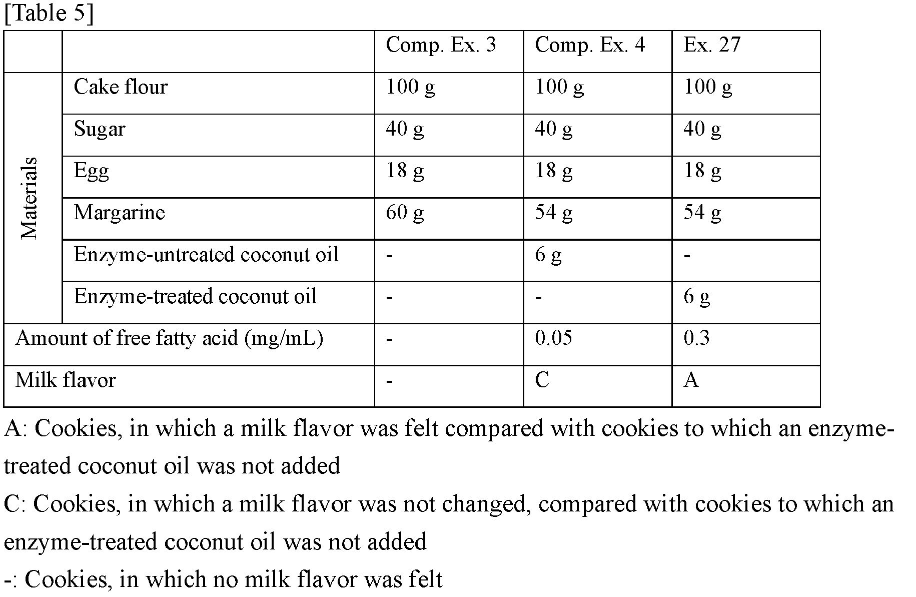

### (3) Consideration

When the enzyme-treated coconut oil was added to actual food products, flavor improvement could be confirmed even with a smaller amount of free fatty acid.

## Claims

1. A flavor-improving enzyme agent for vegetable oils and fats, which comprises lipase.

2. The flavor-improving enzyme agent according to claim 1, wherein the lipase is at least one type selected from the group consisting of a lipase derived from Penicillium sp., a lipase derived from Aspergillus sp., and a lipase derived from Mucor sp.

3. The flavor-improving enzyme agent according to claim 1 or 2, wherein the vegetable oils and fats are coconut oil.

4. A flavor improver for food products, which is obtained by allowing lipase to act on vegetable oils and fats.

5. The flavor improver for food products according to claim 4, wherein the amount of free fatty acid contained in the flavor improver for food products is 0.6 to 500 mg per mL of the vegetable oils and fats.

6. The flavor improver for food products according to claim 4 or 5, wherein the lipase is at least one type selected from the group consisting of a lipase derived from Penicillium sp., a lipase derived from Aspergillus sp., and a lipase derived from Mucor sp.

7. The flavor improver for food products according to any one of claims 4 to 6, wherein the vegetable oils and fats are coconut oil.

8. A food product, comprising the flavor improver for food products according to any one of claims 4 to 7.

9. The food product according to claim 8, wherein the amount of free fatty acid contained in the food product is 0.1 to 60 mg per mL of the vegetable oils and fats.

10. A method for producing a flavor improver for food products, comprising a step of allowing lipase to act on vegetable oils and fats.

11. The method for producing a flavor improver for food products according to claim 10, wherein the lipase is at least one type selected from the group consisting of a lipase derived from Penicillium sp., a lipase derived from Aspergillus sp., and a lipase derived from Mucor sp.

12. The method for producing a flavor improver for food products according to claim 10 or 11, wherein the vegetable oils and fats are coconut oil.

13. The method for producing a flavor improver for food products according to any one of claims 10 to 12, wherein the amount of free fatty acid contained in the flavor improver for food products is 0.6 to 500 mg per mL of the vegetable oils and fats.

14. A method for producing a food product, comprising:
allowing lipase to act on vegetable oils and fats to obtain a flavor improver for food products, and
obtaining a food product comprising the flavor improver for food products.

15. The method for producing a food product according to claim 14, wherein the lipase is at least one type selected from the group consisting of a lipase derived from Penicillium sp., a lipase derived from Aspergillus sp., and a lipase derived from Mucor sp.

16. The method for producing a food product according to claim 14 or 15, wherein the vegetable oils and fats are coconut oil.

17. The method for producing a food product according to any one of claims 14 to 16, wherein the amount of free fatty acid contained in the food product is 0.1 to 60 mg per mL of the vegetable oils and fats.
